Europäisches Patentamt

European Patent Office

Office européen des brevets

(19)

(11) **EP 1 430 873 A1**

(12) **EUROPEAN PATENT APPLICATION**
published in accordance with Art. 158(3) EPC

(43) Date of publication:
 **23.06.2004 Bulletin 2004/26**

(51) Int Cl.⁷: **A61K 7/13**, A61K 7/06,
 A61K 7/09, A61K 7/135

(21) Application number: **02760794.4**

(22) Date of filing: **30.08.2002**

(86) International application number:
 **PCT/JP2002/008853**

(87) International publication number:
 **WO 2003/017959 (06.03.2003 Gazette 2003/10)**

(84) Designated Contracting States:
 **AT BE BG CH CY CZ DE DK EE ES FI FR GB GR
 IE IT LI LU MC NL PT SE SK TR**
 Designated Extension States:
 **AL LT LV MK RO SI**

(30) Priority: **30.08.2001 JP 2001261171**

(71) Applicant: **Nonogawa Shoji Ltd.
 Aichi-ken 460-0002 (JP)**

(72) Inventors:
 • **OSHIMA, Shinji
 Gifu-shi, Gifu 500-8384 (JP)**

• **KATO, Koh
 Toyota-shi, Aichi 471-0062 (JP)**
• **NARUKAWA, Shuji
 Yokkaichi-shi, Mie 510-8006 (JP)**
• **KITO, Naoshi
 Ama-gun, Aichi 490-1202 (JP)**
• **MATSUURA, Jun
 Kasugai-shi, Aichi 480-0304 (JP)**

(74) Representative: **Towler, Philip Dean et al
 Frank B. Dehn & Co.,
 European Patent Attorneys,
 179 Queen Victoria Street
 London EC4V 4EL (GB)**

(54) **HAIR TREATMENT AGENT COMPOSITION AND METHOD FOR PRODUCTION THEREOF**

(57)     There was the problem that users of hair treatment agent compositions have an unpleasant impression during use, because an alkaline agent includes ammonia.

A hair treatment agent composition which comprises an alkaline (A), a base material (B) and a reducing agent (C), which each of the agents and material contains a specific component such as ammonia, an amine, hydrogen sulfide or mercaptan in a mount below the sensitivity of an olfactory organ of a human being. Therefore the hair treatment agent composition devoids a user of unpleasant odor during use. The hair treatment agent composition can be manufactured by a simple process.

Fig.9

| | ammonia | amines | hydrogen sulfide | mercaptan |
|---|---|---|---|---|
| | 1 min, 2 strokes | 1 min, 1 stroke | 1 min, 2 strokes | 1.5 min, 4 strokes |
| | pink → yellow | pink → yellow | light yellow → light brown | yellow → red |
| Example 1  Agent 1 | 1ppm | 0. 5ppm | 0. 05ppm or less | 0. 05ppm or less |
| Example 1  Agent 2 | 0. 2ppm or less | 0. 1ppm or less | 0. 05ppm or less | 0. 05ppm or less |
| Example 1  mixture | 1ppm | 0. 75ppm | 0. 05ppm or less | 0. 05ppm or less |
| Example 2  Agent 1 | 1ppm | 0. 5ppm | 0. 05ppm or less | 0. 05ppm or less |
| Example 2  mixture | 0. 75ppm | 0. 5ppm | 0. 05ppm or less | 0. 05ppm or less |
| Example 3  Agent 1 | 0. 5ppm | 0. 5ppm | 0. 05ppm or less | 0. 05ppm or less |
| Example 3  mixture | 0. 6ppm | 0. 3ppm | 0. 05ppm or less | 0. 05ppm or less |
| Example 4  Agent 1 | 2ppm | 1. 5ppm | 0. 05ppm or less | 0. 05ppm or less |
| Example 4  mixture | 0. 5ppm | 0. 1ppm | 0. 05ppm or less | 0. 05ppm or less |
| Example 5  Agent 1 | 2ppm | 1. 5ppm | 0. 05ppm or less | 0. 05ppm or less |
| Example 5  mixture | 0. 5ppm | 0. 1ppm | 0. 05ppm or less | 0. 05ppm or less |
| Example 6  Agent 1 | 1ppm | 0. 5ppm | 0. 2ppm | 0. 05ppm or less |
| Example 6  Agent 2 | 0. 2ppm | 0. 1ppm | 0. 05ppm or less | 0. 05ppm or less |
| Example 7  Agent 1 | 0. 2ppm | 0. 1ppm | 0. 1ppm | 0. 05ppm or less |
| Example 8  Agent 1 | 1ppm | 0. 5ppm | 0. 4ppm | 0. 05ppm or less |
| | | | | |
| | | | | |
| | | | | |

EP 1 430 873 A1

## Description

### Field of the Invention

The present invention relates to a production method for producing a hair treatment agent composition comprising a plurality of agents constituting hair treatment agents.

### Background Art

Cosmetic products used to treat human keratinous material, such as the scalp hair, body hair, or skin for example, are known to typically contain at least one alkaline agent to bring composition pH to a value of 5 -13. Where a composition has basic pH, the function of the alkaline agent will differ depending on the form of the composition. In the case of a composition employing aqueous hydrogen peroxide as an oxidizing agent, it is necessary to make pH basic in order to decompose the aqueous hydrogen peroxide. Typically, in the case of a composition intended for treatment of scalp hair, making pH basic makes it possible to expand the cuticles of the hair, facilitating penetration of treatment agents into the hair. Where a composition is a depilatory agent, hair dye or bleaching composition, a skin dyeing or depigmenting composition, or a permanent retexturizing composition for the hair for use in permanent wave or hair straightening, with application in many cosmetic compositions, these two actions occur separately or simultaneously.

Alkaline agents typically used in hair treatment compositions of the existing art, for example, scalp hair decolorizing agents and permanent wave compositions, typically have irritating odors, such as aqueous ammonia, ammonium carbonate, ammonium bicarbonate and other ammonium salts. Thus, there was the problem that users of hair treatment agent compositions of this kind have an unpleasant impression during use. For example, aqueous ammonia releases an intense irritating odor that is smothering. Users of permanent wave, dye, or bleaching compositions are particularly inconvenienced. Ammonium carbonate and other ammonium salts also have irritating odors, although not as much as ammonia. With these ammonium salts, in many instances considerable amounts are necessary to give relative highly basic pH, and resultantly, irritating odor on the part of compositions cannot be reduced.

As regards vehicles, those typically used currently emit some kind of vehicle odor. There are some vehicles that have high irritancy to the skin. In the past, it was thought that, as regards such irritating odors and the like, these were unavoidable with hair treatment agent compositions, and the search for countermeasures was limited to ameliorating irritancy through addition of fragrance components and perfumes. It is an object of the present invention to fundamentally solve the problem of irritating odor and the like in hair treatment agent compositions.

## SUMMARY OF THE INVENTION

As a result of intensive research efforts, the inventors perfected the present invention upon discovering that in a hair treatment agent composition comprising a plurality of agents that constitute hair treatment agents, where the plurality of agents certain predetermined components irritant to the olfactory sense are kept at levels below the perceptible threshold of the olfactory sense, there is afforded an odorless type hair treatment agent composition that also produces minimal damage to the hair.

The present invention is directed to hair treatment agent composition comprising a plurality of agents that constitute hair treatment agents: wherein said plurality of agents irritant to the olfactory sense are kept to levels below the perceptible threshold of the olfactory sense. Namely, the hair treatment agent composition of the present invention imparts no unpleasant sensations to the olfactory sense of the user.

The method for producing a hair treatment agent composition comprising a plurality of agents constituting hair treatment agents, the present invention essentially comprises the steps of:

    selecting agents wherein predetermined components irritant to the olfactory sense are present in levels at or below the perceptible threshold of the olfactory sense;
    preparing said plurality of agents; and
    placing said prepared agents in a predetermined receptacle.

According to the production method for a hair treatment agent composition of the present invention, a hair treatment agent composition imparting no unpleasant sensations to the olfactory sense of the user can be produced by means of a simple process.

### BRIEF DESCRIPTION OF THE DRAWINGS

    FIG. 1 is a process diagram showing a production process for a hair treatment agent composition as an embodiment of the present invention;
    FIG. 2 is an illustrative diagram showing the exterior of a representative detector tube;
    FIG. 3 is an illustrative diagram showing the odor measuring method of the Evaluation Benchmark 3 method in an embodiment;
    FIG. 4 is an illustrative diagram showing results of measuring content of four types of component, i.e., ammonia, amines, hydrogen sulfide, and mercaptan, in agents constituting hair treatment agent compositions of the Examples;
    FIG. 5 is an illustrative diagram showing results of measuring content of four types of component, ammonia, amines, hydrogen sulfide, and mercaptan, in other agents constituting hair treatment agent compositions of the Examples;
FIG. 6 is an illustrative diagram showing results with Evaluation Benchmarks 1 and 3 for Examples 1 to 3 and Comparisons 1 to 3;
FIG. 7 is an illustrative diagram showing results with Evaluation Benchmarks 1 and 3 for Examples 4, 5 and Comparisons 4 to 6;
FIG. 8 is an illustrative diagram showing results with Evaluation Benchmarks 1 and 3 for Examples 6 to 8 and Comparisons 7 to 9;
FIG. 9 is an illustrative diagram showing results with Evaluation Benchmark 2 for Examples 1 to 8; and
FIG. 10 is an illustrative diagram showing results with Evaluation Benchmark 2 for Comparisons 1 to 9.

## DESCRIPTION OF THE PREFERRED EMBODIMENTS

The embodiments of the hair treatment agent composition of the present invention are described in detail hereinbelow. When reducing to practice the hair treatment agent composition of the present invention, the plurality of agents are prepared such that levels of predetermined components irritant to the olfactory sense are kept to below the perceptible threshold of the olfactory sense. Each of the plurality of agents may be devoid of perfumes and of components having fragrance. As examples of perfumes, there may be cited fragrant materials such as natural fragrant materials and synthetic fragrant materials, used for the purpose of imparting some sort of fragrance to compositions. As predetermined components producing olfactory stimuli, there are included at least ammonia, amines, hydrogen sulfide, and methyl mercaptan. This is because these components impart unpleasant olfactory sensation to the user. As the plurality of agents, (A) an alkaline agent, (B) a vehicle, (C) a reducing agent and the like may be considered. In preferred practice, by mixing (A) an alkaline agent, (B) a vehicle, and (C) a reducing agent at the time of use, the plurality of agents may be prevented from emitting unpleasant odors irritant to the olfactory sense.
Of the aforementioned plurality of agents, as the alkaline agent of component (A) there may be cited monoethanolamine, diethanolamine, triethanolamine, isopropanolamine, 2-amino-2-methyl-1-propanol, 2-amino-2-methyl-1,3-propanediol, and other such organic amines; arginine and other such basic amino acids; and sodium carbonate, sodium hydroxide, and other such inorganic alkalizing agents. Of these, monoethanolamine is especially preferred.
As the alkaline agent of component (A) there may be used a single kind or combination of two or more kinds, the additive amount thereof preferably being 0.5 -20 wt% of the total composition, and especially preferably 1 -15 wt%. This is because if the additive amount is too small, the effect of swelling the scalp hair will be poor, whereas if too large the action of reducing agents, oxidizing agents etc. may be hindered, and damage to hair and irritancy to skin may become high.
Of these agents, as vehicle (B) there may be cited oils, actives, thickeners, solvents, hair protectants, plant extracts, stabilizers, and all other such existing vehicles that can be typically used. Of these, specific examples of agents that meet the above condition shall be described in the "Examples" hereinbelow, but conversely, materials having a low degree of refinement and some sort of vehicle odor are excluded. For example, oleyl alcohol, oleic acid and oils synthesized therefrom, actives and the like are vehicles having characteristic odor, and are not used as vehicles of the present invention.
As the vehicle of component (B), there are no particular limitations, the material will preferably be highly refined and carefully selected, and in especially preferred practice will be deodorization refined.
As vehicle (B), there may be used a single type, or combination of a greater number, depending on the intended composition application and form etc.; additive amounts thereof can be added in ranges such that the effects of the composition do not suffer, and are not particularly limited.
As the reducing agent of component (C), there may be cited cysteine and its derivatives; thiomalic acid and other mercapto carboxylic acids; thioglycerol and other mercapto alcohols; glycerol monothioglycolate and other mercapto carboxylic acid esters; cysteamine, cisteinamide and other mercapto compounds; sulfurous acid, sodium sulfite, and other sulfites; sodium hydrogensulfite and other hydrogensulfites; and ascorbic acid and its derivatives.
As the reducing agent of component (C), sulfites, hydrogensulfites, ascorbic acid or its derivatives are especially preferred.
As the reducing agent of component (C), there may be used a single kind or combination of two or more kinds, the additive amount thereof preferably being 0.1 -20 wt% of the total composition, and especially preferably 0.5 -15 wt%. This is because if the additive amount is too small, the stability of the composition and its treatment effect on the hair will be poor, whereas if too large damage to hair and irritancy to skin may become high.
The hair treatment agent composition of the present invention may also include active chemical agents. As active chemical agents used in hair treatment agent compositions, there may be cited dye compounds selected from the group consisting of oxidative dye intermediates, couplers, and direct dyes.
As oxidative dye intermediates which are active chemical agents, there may be cited phenylenediamines, aminophenols, diaminopyridines, and salts of these. Of these, p-phenylenediamine, p-toluylenediamine, N, N-bis-(2-hydroxyethyl)-p-phenylenediamine, 2-(2'-hydroxyethyl)-p-phenylenediamine, N-phenyl-p-phenylenediamine, 4,4'-diaminodiphenylamine, 2-chloro-p-phenylenediamine, N, N-dimethyl-p-phenylenediamine, p-aminophenol, o-aminophenol, p-methylaminophenol, 2,-6-dichloro-p-phenylenediamine, p-aminophenylsulfamic acid, 2,5-diaminopyridine, and salts of these, are preferred in terms of effect and hair dyeing power.

Oxidative dye intermediates may be used singly or in combination of two or more types, the additive amount thereof preferably being 0.01 -15 wt%, more preferably 0.1 -10 wt%.

As couplers which are active chemical agents, there may be cited resorcin, pyrogallol, catechol, m-aminophenol, m-phenylenediamine, 2,4-diaminophenol, 1,2,4-benzenetriol, toluene-3,4-diamine, toluene-2,4-diamine, hydroquinone, a-naphthol, 2,6-diaminopyridine, 3,3'-iminodiphenol, 1,5-dihydroxynapthalene, 5-amino-o-cresol, diphenylamine, p-methylaminophenol, fluoroglycine, 2,4-diaminophenoxyethanol, tannic acid, gallic acid, ethyl gallate, methyl gallate, propyl gallate, Japanese gall, 1-methoxy-2-amino-4-(2-hydroxyethyl)aminobenzene, 5-(2-hydroxyamino)-2-methylphenol and salts of these; besides these, those mentioned in "Iyakubu Gaihin Genryo Shikaku" [Quasi-drug Ingredient Standards] (published June 1991, Yakuji Nippo) may be used as appropriate.

Couplers may be used singly or in combination of two or more types, the additive amount thereof preferably being 0.01 - 10 wt%, more preferably 0.1 -5 wt%.

As direct dyes which are active chemical agents, there may be cited nitro dyes, basic dyes, tar based dyes, natural dyes, and other known ones. Of these, nitro based dyes, azo dyes, nitroso dyes, triphenylmethane dyes, xanthene dyes, quinoline dyes, anthraquinone dyes, and indigo dyes are preferred from the standpoint of stability and effect.

Direct dyes may be used singly or in combination of two or more types, the additive amount thereof preferably being 0.001 -15 wt%, more preferably 0.01 -10 wt%.

Next, the embodiments of the production method for a hair treatment agent composition of the present invention are described. In the production method of the present invention, as regards the plurality of agents, agents are selected wherein predetermined components irritant to the olfactory sense are at levels below the perceptible threshold of the olfactory sense; selection at this time should be carried out with the condition that, as predetermined components irritant to the olfactory sense, ammonia, amines, hydrogen sulfide, and methyl mercaptan are all at levels below the perceptible threshold of the olfactory sense. The plurality of agents are prepared, and this is placed in a receptacle; the process may be carried out in the following manner. Specifically, preparation of the preparation may be carried out from a step of grouping the plurality of agents into an oil phase of oil-soluble components, an aqueous phase of water-soluble components, and an additive phase of components that are unstable at high temperature, and weighing these out; a step of separately heating and dissolving said oil phase and aqueous phase components; a step of adding the aqueous phase to the oil phase, each of which has reached above a predetermined temperature, and emulsifying and mixing these; and a step of cooling said emulsified mixture to below a predetermined temperature, and then adding said additive phase to said emulsified mixture at low temperature.

**EXAMPLES**

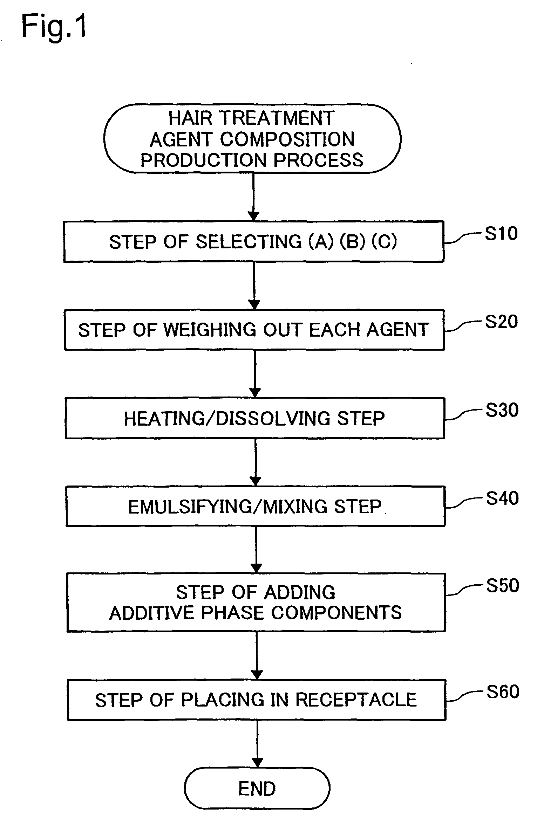

The invention is described in greater detail below on the basis of examples, but the invention is in no way limited thereby. Additive amounts are given in wt%. First, a production method for a hair treatment agent composition of the present invention is described. FIG. 1 is a process diagram showing a production process for a hair treatment agent composition in an example. As shown in the drawing, the composition of the example is produced by a production process comprising

Step S10: where producing a hair treatment agent composition consisting of three types of agent, i.e. (A) an alkaline agent, (B) a vehicle, and (C) a reducing agent, for each of these agents, a step of selecting an agent wherein predetermined components irritant to the olfactory sense are at levels below the perceptible threshold of the olfactory sense,

Step S20: a step of grouping the agents (A) (B) (C) into an oil phase of oil-soluble components, an aqueous phase of water-soluble components, and an additive phase of components that are unstable at high temperature, and weighing these out, Step S30: a step of separately heating and dissolving the oil phase and aqueous phase components,

Step S40: a step of adding the aqueous phase to the oil phase, each of which has reached above a predetermined temperature, and emulsifying mixing these,

Step 550: a step of cooling the emulsified mixture to below a predetermined temperature, and then adding the additive phase components to the emulsified mixture at low temperature, and

Step S60: a step of placing in a predetermined receptacle the emulsified mixture containing the additive phase components.

As hair treatment agent compositions obtained by this process, there are hair dyeing agents, hair decolorizing agents, perming agents and the like. In the aforementioned production process, steps other than Step S10 are known art, and therefore description thereof is omitted; Step S10, namely, for (A) an alkaline agent, (B) a vehicle, and (C) a reducing agent, the step of selecting an agent wherein predetermined components irritant to the olfactory sense are at levels below the perceptible threshold of the olfactory sense, is described in more detail. As predetermined components irritant to the olfactory sense contained in these agents, four types, i.e. ammonia, amines, hydrogen sulfide, and mercaptan, shall be dealt with. These components are typical of components that impart odors to agents includable in hair treatment agent compositions, especially those odors creating an unpleasant sensation for the user. These components, even at content levels on the order of ppm, impart unpleasant sensation to the user, and therefore a detector tube was used for detection thereof.

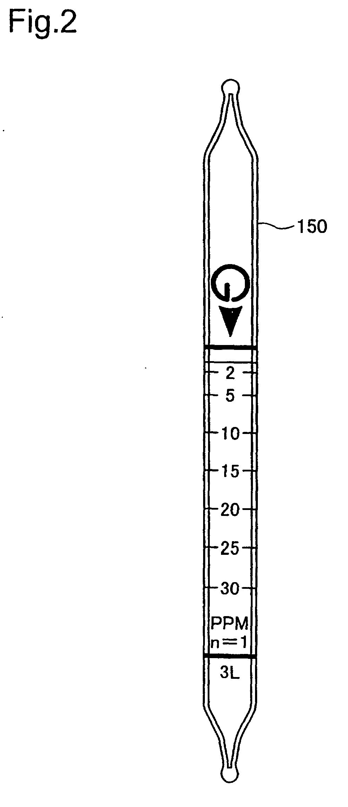

A detector tube has sealed within a substance that reacts only with a particular component and changes color; a typical form is shown in FIG. 2. Detector tube 150 has a predetermined detectable substance and concentration range thereof for each type of tube. For example, with the detector tube called Detector Tube · Ammonia 3 L made by Gastec Corporation, measuring range is 0.5 -78 ppm, detecting limit is 0.2 ppm, and color change is from pink to yellow. This detector tube 150 also has inter alia a predetermined aspiration time for aspiration of gas; with the Ammonia 3L, for example, the number of pump strokes is 2 (100 milliliters), and the time from aspiration to detection is 60 seconds. The detector tube, after this time has elapsed, can determine the concentration of the substance by reading the changed color zone.

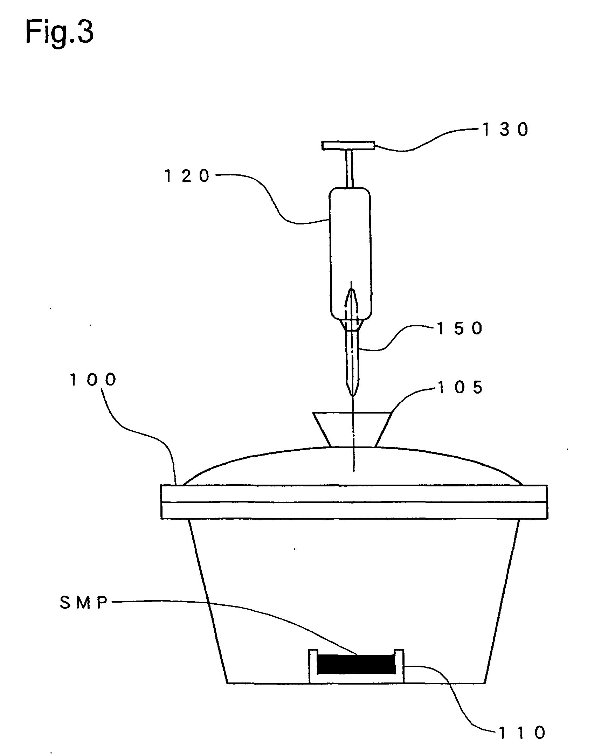

To draw the measurement target into the detector tube 150, as shown in FIG. 3, the procedure of placing 1 gram of measurement target as a sample SMP in a Petri dish 110 that is 30 milli meters in diameter and 15 milli meters deep, and arranged the surface thereof as level as possible, placing this Petri dish 110 in a glass screw top desiccator 100 of 3 liter internal capacity, and hermetically sealing, and

after placing in this state for 3 hours, and with the detection target in the dessicator 100 uniformly dispersed, connecting a detector tube type gas measuring instrument 120 to the screw top, and operating the handle thereof is to aspirate the measuring gas

is performed.

In Step S10, for each agent, selection is performed by measuring concentrations of ammonia, amines, hydrogen sulfide, and mercaptan using dedicated detector tubes. The measurement target gases and detector tube models are as follows.

Ammonia: Gastec Corporation 3L Ammonia Detector Tube

measuring range, 0.5 -78 ppm

detecting limit, 0.2 ppm

2 pump strokes, aspiration amount 100 milliliters per stroke

aspiration time 60 seconds

Amines: Gastec Corporation 180L Amines Detector Tube

measuring range, 0.5 -10 ppm

detecting limit, 0.1 ppm

1 pump stroke, aspiration amount 100 milliliters per stroke

aspiration time 60 seconds

Hydrogen sulfide: Gastec Corporation 4LT Hydrogen Sulfide

Detector Tube

measuring range, 0.1 -4 ppm

detecting limit, 0.05 ppm

2 pump strokes, aspiration amount 100 milliliters per stroke

aspiration time 60 seconds

Mercaptan: Gastec Corporation 70L Mercaptan Detector Tube

measuring range, 0.1 -8.0 ppm

detecting limit, 0.05 ppm

4 pump strokes, aspiration amount 100 milliliters per stroke

aspiration time 90 seconds

Under the said conditions, odor concentrations in each agent which was a measurement target were measured in ppm units. For amines, the Gastec 180 Amines Detector Tube (measuring range, 5 -100 ppm) was used also where needed. This detector tube and the 180L Amines Detector Tube manufactured by the same company have measurement conditions that are otherwise identical.

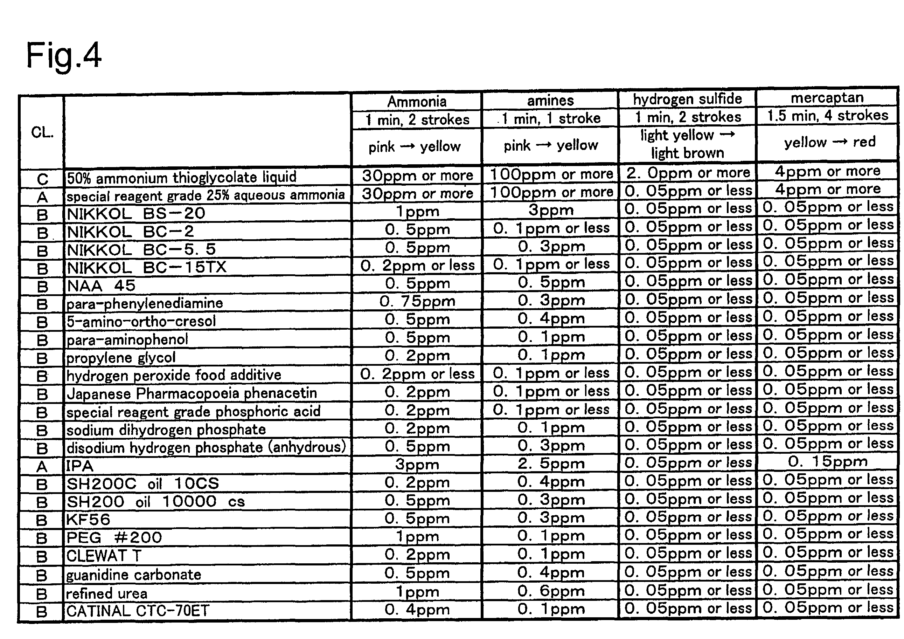

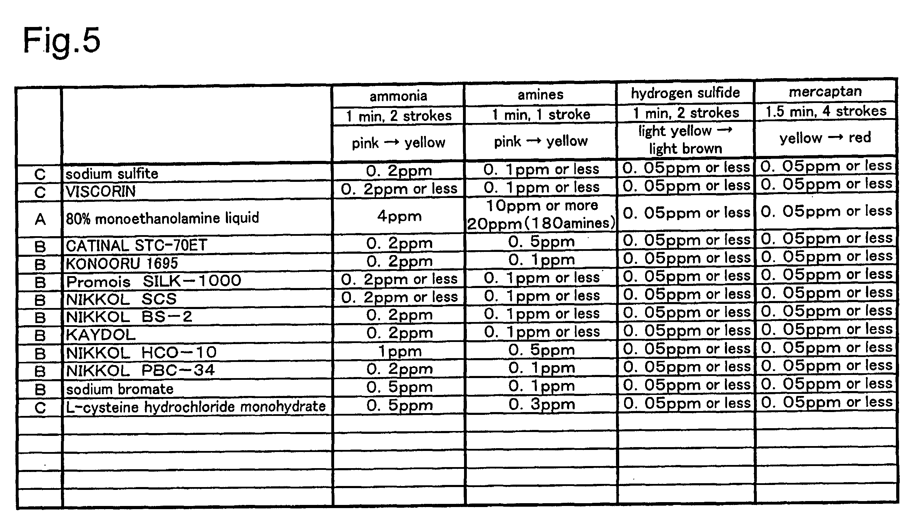

FIGS. 4 and 5 are illustrative diagrams showing, for each of these agents, results of testing, using detector tubes, for content of four types of components components irritant to the olfactory sense, i.e. ammonia, amines, hydrogen sulfide, and mercaptan. The illustrated agents, with the exception of "50% ammonium thioglycolate liquid" "special reagent grade 25% aqueous ammonia", all had concentrations for ammonia, amines, hydrogen sulfide, and mercaptan that were below the perceptible threshold of the olfactory sense. Accordingly, at least two of these agents were not selected for agents of (A) (B) (C). In the column at left in the drawing, components are shown in terms of which of the three types of agent, i.e. (A) alkaline agents, (B) vehicles, and (C) reducing agents, they fall under.

Going through the process described above, by selecting agents of (A) (B) (C) and going through the entire process shown in FIG. 1, a hair treatment agent composition is produced. And so, next, examples of compositions obtained by the said process shall be described next, but prior to description of agents of the examples, the evaluation methods used in the examples shall be described.

<Evaluation Benchmark 1>

[ Odor ] For a hair dyeing agent, hair decolorizing agent, and perming agent, odor perceived by panelists before use (first agent, second agent and/or at the time of mixing first and second agent), during use, and after use was evaluated by expert panelists (10 persons), by means of the following benchmarks.

◎ ... absolutely no perceptible odor

○ ... substantially no perceptible odor

△ ... slight irritating odor or vehicle odor perceptible

X ... intense irritating odor or vehicle odor perceptible

<Evaluation Benchmark 2>

Measurements by detector tubes: Measurements by detector tubes are in principle similar to the aforementioned measurements conducted for the agents (reproduced). Place 1 gram of measurement target as a sample SMP in a Petri dish 110 that is 30 milli meters in diameter and 15 milli meters deep, and arrange the surface thereof as level as possible.

Place this Petri dish 110 in a glass screw top desiccator 100 of 3 liter internal capacity, and hermetically seal.
After placing in this state for 3 hours, and with the detection target in the dessicator 100 uniformly dispersed, connect a detector tube type gas measuring instrument 120 to the screw top, and operate the handle thereof is to aspirate the measuring gas, and measure ammonia, amines, hydrogen sulfide, and mercaptan concentrations using dedicated detector tubes.

The measurement target gases and detector tube models are as follows (reproduced).
Ammonia: Gastec Corporation 3L Ammonia Detector Tube

measuring range, 0.5 -78 ppm

detecting limit, 0.2 ppm

2 pump strokes, aspiration amount 100 milliliters per stroke

aspiration time 60 seconds

Amines: Gastec Corporation 180L Amines Detector Tube

measuring range, 0.5 -10 ppm

detecting limit, 0.1 ppm

1 pump stroke, aspiration amount 100 milliliters per stroke

aspiration time 60 seconds

Hydrogen sulfide: Gastec Corporation 4LT Hydrogen Sulfide

Detector Tube

measuring range, 0.1 -4 ppm

detecting limit, 0.05 ppm

2 pump strokes, aspiration amount 100 milliliters per stroke

aspiration time 60 seconds

Mercaptan: Gastec Corporation 70L Mercaptan Detector Tube

measuring range, 0.1 -8.0 ppm

detecting limit, 0.05 ppm

4 pump strokes, aspiration amount 100 milliliters per stroke

aspiration time 90 seconds

Under the said conditions, odor concentrations in each agent which was a measurement target were measured in ppm units.

<Evaluation Benchmark 3>

[ Damage to Scalp Hair ]

30 strands of hair each were drawn from bundles of hair treated with a hair decolorizing agent, and perming agent, two end portions 5 cm and 6 cm from the hair ends were held with glass fiber-containing tape 1 cm wide, breakage was produced at the 5 cm portion and 6 cm portion from the hair ends, the diameter of the hair was measured with a micrometer, the breaking weight was measured with a rheometer, and the tensile strength of the hair was calculated using Equation (1) below.

$$tensile\ strength(kg\ /\ mm^2) = \frac{A/1000}{\pi(B/2)^2} \qquad (1)$$

A: breaking weight (g)
B: hair diameter (mm)

<Examples 1 -3, Comparisons 1 -3>

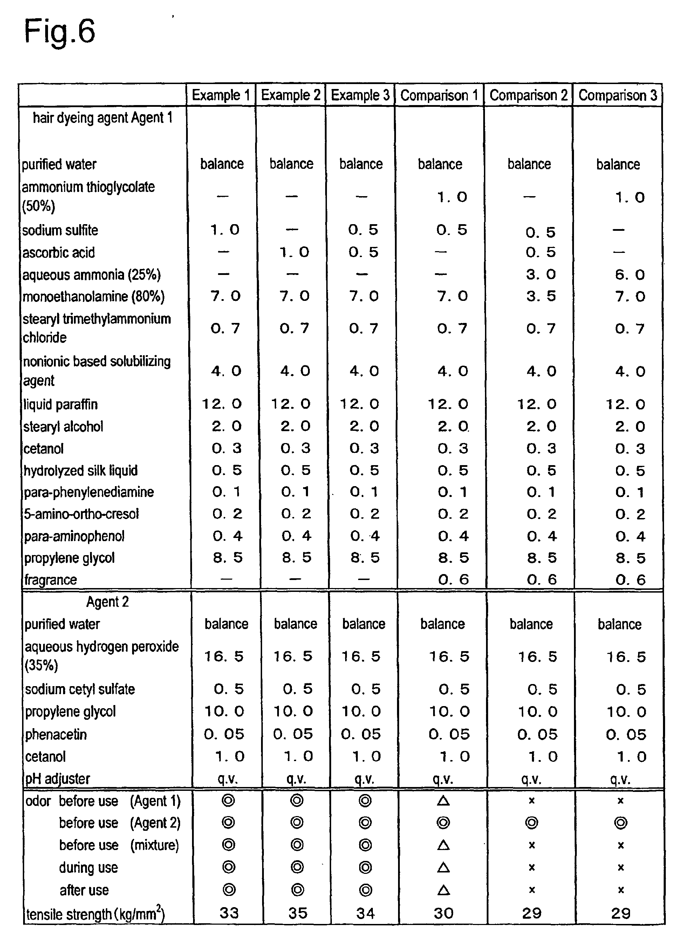

Formulations of Agent 1 and Agent 2 in Examples 1 to 3 and Comparisons 1 -3, evaluations of odor by means of Evaluation Benchmark 1, and evaluations of damage to hair by means of Evaluation Benchmark 3 are shown in FIG. 6. In FIG. 6, each component was prepared as a hair dyeing agent composition Agent 1 and Agent 2 sample using the process shown in FIG. 1.

In the Examples, the "nonionic based solubilizing agent" was polyoxyethylene cetyl ether obtainable as the "BC Series" manufactured by Nikko Chemicals. Substitute products would include polyoxyethylene stearyl ether from the "BC Series" by the same company, the "EMALEX 600 Series" manufactured by Nihon Emulsion Co. Ltd. and the like. As the "hydrolyzed silk liquid", "Promois SILK-1000" manufactured by Seiwa Kasei Co. Ltd. was used. Additionally, as a fragrance material, a 2 : 1 : 1 : 2 mixture of natural essential oils, i.e., components extracted from orange, lemon, bergamot, and mandarin, was used. Of these, Examples 4, 5 and Comparisons 4 to 6 described hereinbelow are the same as regards the nonionic based solubilizing agent. As regards the hydrolyzed silk liquid and fragrance material, the same ones were used in all of the Examples and all of the Comparisons.

Next, Agent 1 and Agent 2 were mixed in a respective weight ratio of 1 : 2. In FIG. 6, (mixture) indicates this mixed condition. The mixed material was applied to untreated hair 20 cm in length weighing15 g, left for 30 minutes, and then rinsed with water, then dried with a dryer. Tensile strength as per Evaluation Benchmark 3 was measured in this state.

As shown in the drawing, with Examples 1 to 3, panelists perceived absolutely no odor on the part of Agent 1, Agent 2, or mixture of the two. Tensile strength of hair subjected to treatment with the compositions of the Examples was 33 kilograms or above in each case, and damage was negligible. In contrast to this, with the Comparisons, either slight odor (Comparison 1) or intense odor was perceived on the part of Agent 1 and when the two agents were mixed. Tensile strength was 30 kilograms or lower.

<Examples 4, 5, Comparisons 4 -6>

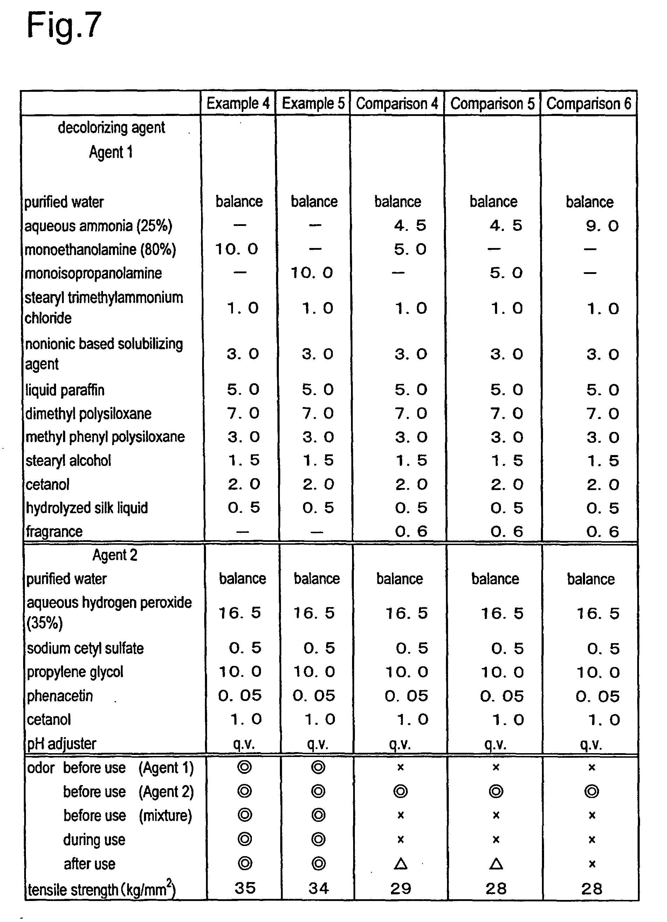

Formulations of Agent 1 and Agent 2 in Examples 4, 5 and Comparisons 4 to 6, evaluations of odor by means of Evaluation Benchmark 1, and evaluations of damage to hair by means of Evaluation Benchmark 3 are shown in FIG. 7. In FIG. 7, each component was prepared as a hair decolorizing agent composition Agent 1 and Agent 2 sample using the process shown in FIG. 1.

Next, Agent 1 and Agent 2 were mixed in a respective weight ratio of 1 : 2. In FIG. 7, (mixture) indicates this mixed condition. The mixed material was applied to untreated hair 20 cm in length weighing 15 g, left for 30 minutes, and then rinsed with water, then dried with a dryer. Tensile strength as per Evaluation Benchmark 3 was measured in this state.

As shown in the drawing, with Examples 4, 5, panelists perceived absolutely no odor on the part of Agent 1, Agent 2, or mixture of the two. Tensile strength of hair subjected to treatment with the compositions of the Examples was 34 kilograms or above in each case, and damage was negligible. In contrast to this, with the Comparisons, intense odor was perceived on the part of Agent 1 and when the two agents were mixed. Tensile strength was 29 kilograms or lower.

<Examples 6 -8, Comparisons 7 -9>

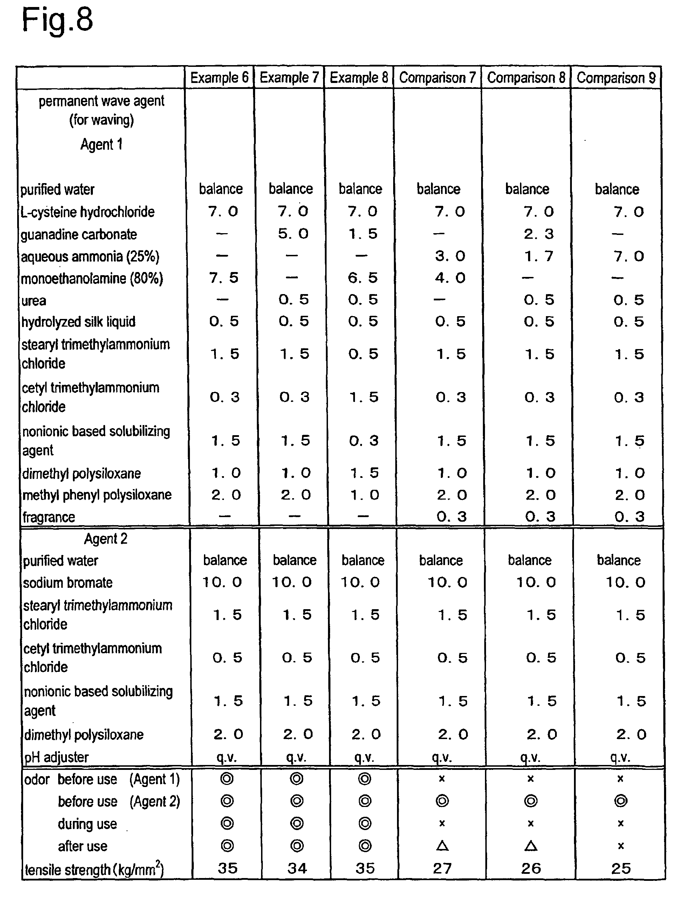

Formulations of Agent 1 and Agent 2 in Examples 6 to 8 and Comparisons 7 to 9, evaluations of odor by means of Evaluation Benchmark 1, and evaluations of damage to hair by means of Evaluation Benchmark 3 are shown in FIG. 8. In FIG. 8, each component was prepared as a permanent wave composition Agent 1 and Agent 2 sample using the process shown in FIG. 1.

In Examples 6 to 8 and Comparisons 7 to 9, the "nonionic based solubilizing agent" was polyoxyethylene castor oil (or hydrogenated castor oil) obtainable as the "CO (or HCO) Series" manufactured by Nikko Chemicals. Substitute products would include polyoxyethylene polyoxypropylene alkyl ethers from the "PBC (or PEN) Series" by the same company.

Next, untreated hair 20 cm in length weighing 15 g, was coiled around a rod 13 mm in diameter, immersed for 10 minutes in permanent wave Agent 1 shown in FIG. 8, rinsed in water, and dried with a dryer. Tensile strength as per Evaluation Benchmark 3 was measured in this state.

As shown in the drawing, with Examples 6 to 8 5, panelists perceived absolutely no odor on the part of Agent 1 or Agent 2, either during use or after use. Tensile strength of hair subjected to treatment with the compositions of the Examples was 34 kilograms or above in each case, and damage was negligible. In contrast to this, with the Comparisons, intense odor was perceived on the part of Agent 1 alone, and both during and after use. Tensile strength was 25 kilograms or lower.

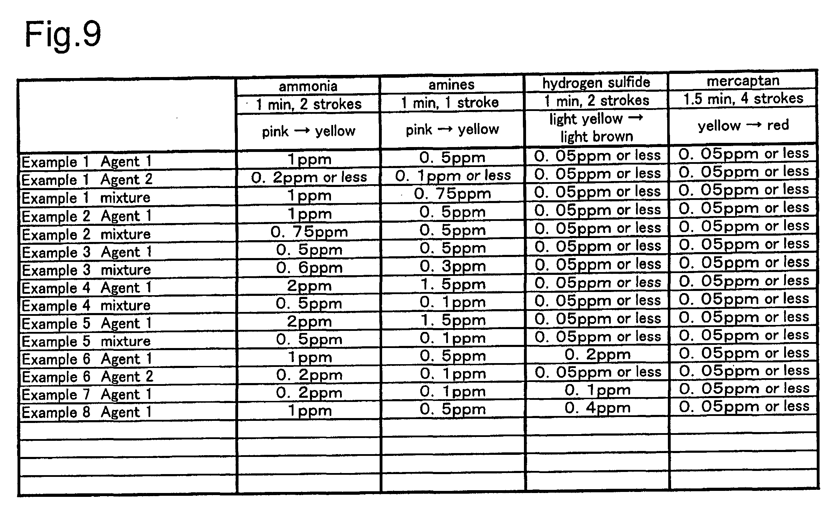

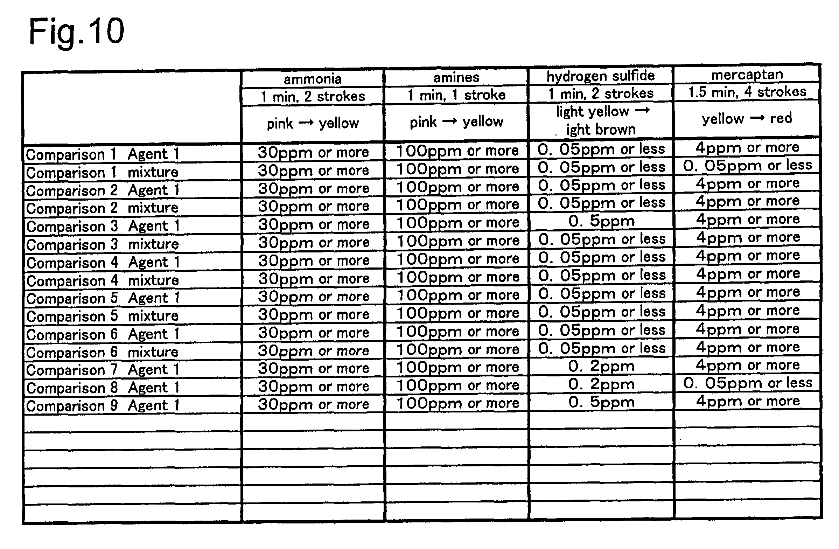

Results of odor evaluation per Evaluation Benchmark 2 for the aforementioned Examples 1 to 8 are given in FIG. 9. In the drawing, with the exception of Example 1 and Example 6, Agent 2 is not given in the table; however, as regards Example 2 to Example 5, Agent 2 is the same as in Example 1, and as regards Examples 7, 8, it is the same as in Example 6, so respective results therefor have been omitted from the table. For the aforementioned Comparisons 1 to 9, results measured in similar fashion are shown in FIG. 10.

For Examples 1 to 8, as shown in FIG. 9, it will be apparent that ammonia, amines, hydrogen sulfide, and mercaptan were all substantially undetected, or present in extremely trace amounts, and that unpleasant odor based on these components was substantially absent. On the other hand, with the Comparisons shown in FIG. 10, ammonia was =30 ppm (an amount in excess of the upper limit of detection of the detector tube used), amines were =100 ppm (an amount in excess of the upper limit of detection of the detector tube used), and mercaptan, with the exception of the mixed material in Comparison 1 and Agent 1 in Comparison 8, was detected at =4 ppm, from which it will be apparent that the user would perceive a fairly intense unpleasant odor.

## EFFECT OF THE INVENTION

According to the hair treatment agent composition of the present invention set forth in detail hereinabove, compositions per se are devoid of unpleasant odors. Accordingly, the user can use hair treatment agent compositions of various kinds without concern about unpleasant odors. Further, according to the hair treatment agent composition production method of the present invention, hair treatment agent compositions devoid of unpleasant odor during use can be manufactured by a simple process.

**Industrial Applicability**

The hair treatment agent composition of the present invention is applicable for hair dye or bleaching composition or a permanent retexturizing composition for the hair for use in permanent wave.

**Claims**

**1.** Hair treatment agent composition comprising a plurality of agents that constitute hair treatment agents:

wherein said plurality of agents irritant to the olfactory sense are kept to levels below the perceptible threshold of the olfactory sense.

2. Hair treatment agent composition according to claim 1, wherein each of said plurality of agents is devoid of perfumes and of components having fragrance.

3. Hair treatment agent composition according to claim 1, wherein said predetermined components producing olfactory stimuli include at least ammonia, amines, hydrogen sulfide, and methyl mercaptan.

4. Hair treatment agent composition according to claim 1, wherein said plurality of agents are an alkaline agent(A), a vehicle(B), and a reducing agent (C),

said an alkaline agent(A), a vehicle (B) , and a reducing agent (C) being mixed at the time of use to prevent emission of unpleasant odors irritant to the olfactory sense.

5. Hair treatment agent composition according to claim 4, wherein said alkaline agent (A) includes at least one compound selected from the group consisting of monoethanolamine, diethanolamine, triethanolamine, isopropanolamine, 2-amino-2-methyl-1-propanol, 2-amino-2-methyl-1,3-propanediol, and other such organic amines; arginine and other such basic amino acids; and sodium carbonate, sodium hydroxide, and other such inorganic alkalizing agents.

6. Hair treatment agent composition according to claim 4 wherein said vehicle (B) is a deodorization refined, highly refined material.

7. Hair treatment agent composition according to claim 4 wherein said reducing agent (C) includes at least one compound selected from the group consisting of cysteine and its derivatives; thiomalic acid and other mercapto carboxylic acids; thioglycerol and other mercapto alcohols; glycerol monothioglycolate and other mercapto carboxylic acid esters; cysteamine, cisteinamide and other mercapto compounds; sulfurous acid, sodium sulfite, and other sulfites; sodium hydrogensulfite and other hydrogensulfites; and ascorbic acid and its derivatives.

8. Hair treatment agent composition according to anyone of claims 1 to 7, which is a composition for use in dyeing or decolorizing the hair, wherein pH of said composition is in the range 7 -13.

9. Hair treatment agent composition according to anyone of claims 1 to 7, containing as an active conditioner at least one dye compound selected from the group consisting of oxidative dye intermediates, couplers, and direct dyes.

10. Hair treatment agent composition according to claim 9, having composition pH within the range 5 -13.

11. Hair treatment agent composition according to anyone of claims 1 to 7, which is a composition used for permanent retexturizing of the hair, wherein pH of said composition is in the range 5 -10.

12. Production method for a hair treatment agent composition comprising a plurality of hair treatment agents, the method comprising the steps of

for said plurality of agents, selecting agents wherein predetermined components irritant to the olfactory sense [are present] in levels at or below the perceptible threshold of the olfactory sense;

preparing said plurality of agents; and

placing said prepared agents in a predetermined receptacle.

13. Production method for a hair treatment agent composition according to claim 12, wherein said plurality of agents are an alkaline agent (A) , a vehicle (B) , and a reducing agent (C) , and prepared by blending the other agents with at least vehicle (B).

14. Production method for a hair treatment agent composition according to claim 12, wherein selection of said agents is performed on the condition that as predetermined components producing olfactory stimuli, ammonia, amines, hydrogen sulfide, and methyl mercaptan each are below the perceptible threshold of the olfactory sense.

15. Production method for a hair treatment agent composition according to claim 12 or 14,

wherein preparation of said preparation comprises:

a step of grouping said plurality of agents into an oil phase of oil-soluble components, an aqueous phase of water-soluble components, and an additive phase of components that are unstable at high temperature, and weighing these out;

a step of separately heating and dissolving said oil phase and aqueous phase components;

a step of adding said aqueous phase to said oil phase, each of which has reached above a predetermined temperature, and emulsifying and mixing these; and

a step of cooling said emulsified mixture to below a predetermined temperature, and then adding said additive phase to said emulsified mixture at low temperature.

## Fig.1

```
     ┌─────────────────────────┐
     │     HAIR TREATMENT      │
     │   AGENT COMPOSITION     │
     │   PRODUCTION PROCESS    │
     └─────────────────────────┘
                 │
                 ▼
   ┌───────────────────────────────┐
   │  STEP OF SELECTING (A) (B) (C) │──── S10
   └───────────────────────────────┘
                 │
                 ▼
   ┌───────────────────────────────┐
   │  STEP OF WEIGHING OUT EACH AGENT │──── S20
   └───────────────────────────────┘
                 │
                 ▼
   ┌───────────────────────────────┐
   │   HEATING/DISSOLVING STEP     │──── S30
   └───────────────────────────────┘
                 │
                 ▼
   ┌───────────────────────────────┐
   │   EMULSIFYING/MIXING STEP     │──── S40
   └───────────────────────────────┘
                 │
                 ▼
   ┌───────────────────────────────┐
   │       STEP OF ADDING          │──── S50
   │   ADDITIVE PHASE COMPONENTS   │
   └───────────────────────────────┘
                 │
                 ▼
   ┌───────────────────────────────┐
   │  STEP OF PLACING IN RECEPTACLE │──── S60
   └───────────────────────────────┘
                 │
                 ▼
            ┌─────────┐
            │   END   │
            └─────────┘
```

# Fig.2

Fig.3

# Fig.4

| CL. | | Ammonia 1 min, 2 strokes pink → yellow | amines 1 min, 1 stroke pink → yellow | hydrogen sulfide 1 min, 2 strokes light yellow → light brown | mercaptan 1.5 min, 4 strokes yellow → red |
|---|---|---|---|---|---|
| C | 50% ammonium thioglycolate liquid | 30ppm or more | 100ppm or more | 2. 0ppm or more | 4ppm or more |
| A | special reagent grade 25% aqueous ammonia | 30ppm or more | 100ppm or more | 0. 05ppm or less | 4ppm or more |
| B | NIKKOL BS−20 | 1ppm | 3ppm | 0. 05ppm or less | 0. 05ppm or less |
| B | NIKKOL BC−2 | 0. 5ppm | 0. 1ppm or less | 0. 05ppm or less | 0. 05ppm or less |
| B | NIKKOL BC−5. 5 | 0. 5ppm | 0. 3ppm | 0. 05ppm or less | 0. 05ppm or less |
| B | NIKKOL BC−15TX | 0. 2ppm or less | 0. 1ppm or less | 0. 05ppm or less | 0. 05ppm or less |
| B | NAA 45 | 0. 5ppm | 0. 5ppm | 0. 05ppm or less | 0. 05ppm or less |
| B | para-phenylenediamine | 0. 75ppm | 0. 3ppm | 0. 05ppm or less | 0. 05ppm or less |
| B | 5-amino-ortho-cresol | 0. 5ppm | 0. 4ppm | 0. 05ppm or less | 0. 05ppm or less |
| B | para-aminophenol | 0. 5ppm | 0. 1ppm | 0. 05ppm or less | 0. 05ppm or less |
| B | propylene glycol | 0. 2ppm | 0. 1ppm | 0. 05ppm or less | 0. 05ppm or less |
| B | hydrogen peroxide food additive | 0. 2ppm or less | 0. 1ppm or less | 0. 05ppm or less | 0. 05ppm or less |
| B | Japanese Pharmacopoeia phenacetin | 0. 2ppm | 0. 1ppm or less | 0. 05ppm or less | 0. 05ppm or less |
| B | special reagent grade phosphoric acid | 0. 2ppm | 0. 1ppm or less | 0. 05ppm or less | 0. 05ppm or less |
| B | sodium dihydrogen phosphate | 0. 2ppm | 0. 1ppm | 0. 05ppm or less | 0. 05ppm or less |
| B | disodium hydrogen phosphate (anhydrous) | 0. 5ppm | 0. 3ppm | 0. 05ppm or less | 0. 05ppm or less |
| A | IPA | 3ppm | 2. 5ppm | 0. 05ppm or less | 0. 15ppm |
| B | SH200C oil 10CS | 0. 2ppm | 0. 4ppm | 0. 05ppm or less | 0. 05ppm or less |
| B | SH200 oil 10000 cs | 0. 5ppm | 0. 3ppm | 0. 05ppm or less | 0. 05ppm or less |
| B | KF56 | 0. 5ppm | 0. 3ppm | 0. 05ppm or less | 0. 05ppm or less |
| B | PEG #200 | 1ppm | 0. 1ppm | 0. 05ppm or less | 0. 05ppm or less |
| B | CLEWAT T | 0. 2ppm | 0. 1ppm | 0. 05ppm or less | 0. 05ppm or less |
| B | guanidine carbonate | 0. 5ppm | 0. 4ppm | 0. 05ppm or less | 0. 05ppm or less |
| B | refined urea | 1ppm | 0. 6ppm | 0. 05ppm or less | 0. 05ppm or less |
| B | CATINAL CTC-70ET | 0. 4ppm | 0. 1ppm | 0. 05ppm or less | 0. 05ppm or less |

# Fig.5

| | | ammonia | amines | hydrogen sulfide | mercaptan |
|---|---|---|---|---|---|
| | | 1 min, 2 strokes | 1 min, 1 stroke | 1 min, 2 strokes | 1.5 min, 4 strokes |
| | | pink → yellow | pink → yellow | light yellow → light brown | yellow → red |
| C | sodium sulfite | 0. 2ppm | 0. 1ppm or less | 0. 05ppm or less | 0. 05ppm or less |
| C | VISCORIN | 0. 2ppm or less | 0. 1ppm or less | 0. 05ppm or less | 0. 05ppm or less |
| A | 80% monoethanolamine liquid | 4ppm | 10ppm or more 20ppm(180amines) | 0. 05ppm or less | 0. 05ppm or less |
| B | CATINAL STC-70ET | 0. 2ppm | 0. 5ppm | 0. 05ppm or less | 0. 05ppm or less |
| B | KONOORU 1695 | 0. 2ppm | 0. 1ppm | 0. 05ppm or less | 0. 05ppm or less |
| B | Promois SILK-1000 | 0. 2ppm or less | 0. 1ppm or less | 0. 05ppm or less | 0. 05ppm or less |
| B | NIKKOL SCS | 0. 2ppm or less | 0. 1ppm or less | 0. 05ppm or less | 0. 05ppm or less |
| B | NIKKOL BS-2 | 0. 2ppm | 0. 1ppm or less | 0. 05ppm or less | 0. 05ppm or less |
| B | KAYDOL | 0. 2ppm | 0. 1ppm or less | 0. 05ppm or less | 0. 05ppm or less |
| B | NIKKOL HCO-10 | 1ppm | 0. 5ppm | 0. 05ppm or less | 0. 05ppm or less |
| B | NIKKOL PBC-34 | 0. 2ppm | 0. 1ppm | 0. 05ppm or less | 0. 05ppm or less |
| B | sodium bromate | 0. 5ppm | 0. 1ppm | 0. 05ppm or less | 0. 05ppm or less |
| C | L-cysteine hydrochloride monohydrate | 0. 5ppm | 0. 3ppm | 0. 05ppm or less | 0. 05ppm or less |
| | | | | | |
| | | | | | |
| | | | | | |
| | | | | | |

# Fig.6

| | Example 1 | Example 2 | Example 3 | Comparison 1 | Comparison 2 | Comparison 3 |
|---|---|---|---|---|---|---|
| hair dyeing agent Agent 1 | | | | | | |
| purified water | balance | balance | balance | balance | balance | balance |
| ammonium thioglycolate (50%) | — | — | — | 1. 0 | — | 1. 0 |
| sodium sulfite | 1. 0 | — | 0. 5 | 0. 5 | 0. 5 | — |
| ascorbic acid | — | 1. 0 | 0. 5 | — | 0. 5 | — |
| aqueous ammonia (25%) | — | — | — | — | 3. 0 | 6. 0 |
| monoethanolamine (80%) | 7. 0 | 7. 0 | 7. 0 | 7. 0 | 3. 5 | 7. 0 |
| stearyl trimethylammonium chloride | 0. 7 | 0. 7 | 0. 7 | 0. 7 | 0. 7 | 0. 7 |
| nonionic based solubilizing agent | 4. 0 | 4. 0 | 4. 0 | 4. 0 | 4. 0 | 4. 0 |
| liquid paraffin | 12. 0 | 12. 0 | 12. 0 | 12. 0 | 12. 0 | 12. 0 |
| stearyl alcohol | 2. 0 | 2. 0 | 2. 0 | 2. 0 | 2. 0 | 2. 0 |
| cetanol | 0. 3 | 0. 3 | 0. 3 | 0. 3 | 0. 3 | 0. 3 |
| hydrolyzed silk liquid | 0. 5 | 0. 5 | 0. 5 | 0. 5 | 0. 5 | 0. 5 |
| para-phenylenediamine | 0. 1 | 0. 1 | 0. 1 | 0. 1 | 0. 1 | 0. 1 |
| 5-amino-ortho-cresol | 0. 2 | 0. 2 | 0. 2 | 0. 2 | 0. 2 | 0. 2 |
| para-aminophenol | 0. 4 | 0. 4 | 0. 4 | 0. 4 | 0. 4 | 0. 4 |
| propylene glycol | 8. 5 | 8. 5 | 8. 5 | 8. 5 | 8. 5 | 8. 5 |
| fragrance | — | — | — | 0. 6 | 0. 6 | 0. 6 |
| Agent 2 | | | | | | |
| purified water | balance | balance | balance | balance | balance | balance |
| aqueous hydrogen peroxide (35%) | 16. 5 | 16. 5 | 16. 5 | 16. 5 | 16. 5 | 16. 5 |
| sodium cetyl sulfate | 0. 5 | 0. 5 | 0. 5 | 0. 5 | 0. 5 | 0. 5 |
| propylene glycol | 10. 0 | 10. 0 | 10. 0 | 10. 0 | 10. 0 | 10. 0 |
| phenacetin | 0. 05 | 0. 05 | 0. 05 | 0. 05 | 0. 05 | 0. 05 |
| cetanol | 1. 0 | 1. 0 | 1. 0 | 1. 0 | 1. 0 | 1. 0 |
| pH adjuster | q.v. | q.v. | q.v. | q.v. | q.v. | q.v. |
| odor  before use  (Agent 1) | ◎ | ◎ | ◎ | △ | × | × |
| before use  (Agent 2) | ◎ | ◎ | ◎ | ◎ | ◎ | ◎ |
| before use  (mixture) | ◎ | ◎ | ◎ | △ | × | × |
| during use | ◎ | ◎ | ◎ | △ | × | × |
| after use | ◎ | ◎ | ◎ | △ | × | × |
| tensile strength (kg/mm$^2$) | 33 | 35 | 34 | 30 | 29 | 29 |

# Fig.7

| | Example 4 | Example 5 | Comparison 4 | Comparison 5 | Comparison 6 |
|---|---|---|---|---|---|
| decolorizing agent<br>Agent 1 | | | | | |
| purified water | balance | balance | balance | balance | balance |
| aqueous ammonia (25%) | — | — | 4. 5 | 4. 5 | 9. 0 |
| monoethanolamine (80%) | 10. 0 | — | 5. 0 | — | — |
| monoisopropanolamine | — | 10. 0 | — | 5. 0 | — |
| stearyl trimethylammonium chloride | 1. 0 | 1. 0 | 1. 0 | 1. 0 | 1. 0 |
| nonionic based solubilizing agent | 3. 0 | 3. 0 | 3. 0 | 3. 0 | 3. 0 |
| liquid paraffin | 5. 0 | 5. 0 | 5. 0 | 5. 0 | 5. 0 |
| dimethyl polysiloxane | 7. 0 | 7. 0 | 7. 0 | 7. 0 | 7. 0 |
| methyl phenyl polysiloxane | 3. 0 | 3. 0 | 3. 0 | 3. 0 | 3. 0 |
| stearyl alcohol | 1. 5 | 1. 5 | 1. 5 | 1. 5 | 1. 5 |
| cetanol | 2. 0 | 2. 0 | 2. 0 | 2. 0 | 2. 0 |
| hydrolyzed silk liquid | 0. 5 | 0. 5 | 0. 5 | 0. 5 | 0. 5 |
| fragrance | — | — | 0. 6 | 0. 6 | 0. 6 |
| Agent 2 | | | | | |
| purified water | balance | balance | balance | balance | balance |
| aqueous hydrogen peroxide (35%) | 16. 5 | 16. 5 | 16. 5 | 16. 5 | 16. 5 |
| sodium cetyl sulfate | 0. 5 | 0. 5 | 0. 5 | 0. 5 | 0. 5 |
| propylene glycol | 10. 0 | 10. 0 | 10. 0 | 10. 0 | 10. 0 |
| phenacetin | 0. 05 | 0. 05 | 0. 05 | 0. 05 | 0. 05 |
| cetanol | 1. 0 | 1. 0 | 1. 0 | 1. 0 | 1. 0 |
| pH adjuster | q.v. | q.v. | q.v. | q.v. | q.v. |
| odor before use (Agent 1) | ◎ | ◎ | × | × | × |
| before use (Agent 2) | ◎ | ◎ | ◎ | ◎ | ◎ |
| before use (mixture) | ◎ | ◎ | × | × | × |
| during use | ◎ | ◎ | × | × | × |
| after use | ◎ | ◎ | △ | △ | × |
| tensile strength (kg/mm$^2$) | 35 | 34 | 29 | 28 | 28 |

# Fig.8

| | Example 6 | Example 7 | Example 8 | Comparison 7 | Comparison 8 | Comparison 9 |
|---|---|---|---|---|---|---|
| permanent wave agent (for waving) Agent 1 | | | | | | |
| purified water | balance | balance | balance | balance | balance | balance |
| L-cysteine hydrochloride | 7. 0 | 7. 0 | 7. 0 | 7. 0 | 7. 0 | 7. 0 |
| guanadine carbonate | — | 5. 0 | 1. 5 | — | 2. 3 | — |
| aqueous ammonia (25%) | — | — | — | 3. 0 | 1. 7 | 7. 0 |
| monoethanolamine (80%) | 7. 5 | — | 6. 5 | 4. 0 | — | — |
| urea | — | 0. 5 | 0. 5 | — | 0. 5 | 0. 5 |
| hydrolyzed silk liquid | 0. 5 | 0. 5 | 0. 5 | 0. 5 | 0. 5 | 0. 5 |
| stearyl trimethylammonium chloride | 1. 5 | 1. 5 | 0. 5 | 1. 5 | 1. 5 | 1. 5 |
| cetyl trimethylammonium chloride | 0. 3 | 0. 3 | 1. 5 | 0. 3 | 0. 3 | 0. 3 |
| nonionic based solubilizing agent | 1. 5 | 1. 5 | 0. 3 | 1. 5 | 1. 5 | 1. 5 |
| dimethyl polysiloxane | 1. 0 | 1. 0 | 1. 5 | 1. 0 | 1. 0 | 1. 0 |
| methyl phenyl polysiloxane | 2. 0 | 2. 0 | 1. 0 | 2. 0 | 2. 0 | 2. 0 |
| fragrance | — | — | — | 0. 3 | 0. 3 | 0. 3 |
| Agent 2 | | | | | | |
| purified water | balance | balance | balance | balance | balance | balance |
| sodium bromate | 10. 0 | 10. 0 | 10. 0 | 10. 0 | 10. 0 | 10. 0 |
| stearyl trimethylammonium chloride | 1. 5 | 1. 5 | 1. 5 | 1. 5 | 1. 5 | 1. 5 |
| cetyl trimethylammonium chloride | 0. 5 | 0. 5 | 0. 5 | 0. 5 | 0. 5 | 0. 5 |
| nonionic based solubilizing agent | 1. 5 | 1. 5 | 1. 5 | 1. 5 | 1. 5 | 1. 5 |
| dimethyl polysiloxane | 2. 0 | 2. 0 | 2. 0 | 2. 0 | 2. 0 | 2. 0 |
| pH adjuster | q.v. | q.v. | q.v. | q.v. | q.v. | q.v. |
| odor before use (Agent 1) | ◎ | ◎ | ◎ | × | × | × |
| before use (Agent 2) | ◎ | ◎ | ◎ | ◎ | ◎ | ◎ |
| during use | ◎ | ◎ | ◎ | × | × | × |
| after use | ◎ | ◎ | ◎ | △ | △ | × |
| tensile strength $(kg/mm^2)$ | 35 | 34 | 35 | 27 | 26 | 25 |

# Fig.9

| | ammonia | amines | hydrogen sulfide | mercaptan |
|---|---|---|---|---|
| | 1 min, 2 strokes | 1 min, 1 stroke | 1 min, 2 strokes | 1.5 min, 4 strokes |
| | pink → yellow | pink → yellow | light yellow → light brown | yellow → red |
| Example 1 Agent 1 | 1ppm | 0. 5ppm | 0. 05ppm or less | 0. 05ppm or less |
| Example 1 Agent 2 | 0. 2ppm or less | 0. 1ppm or less | 0. 05ppm or less | 0. 05ppm or less |
| Example 1 mixture | 1ppm | 0. 75ppm | 0. 05ppm or less | 0. 05ppm or less |
| Example 2 Agent 1 | 1ppm | 0. 5ppm | 0. 05ppm or less | 0. 05ppm or less |
| Example 2 mixture | 0. 75ppm | 0. 5ppm | 0. 05ppm or less | 0. 05ppm or less |
| Example 3 Agent 1 | 0. 5ppm | 0. 5ppm | 0. 05ppm or less | 0. 05ppm or less |
| Example 3 mixture | 0. 6ppm | 0. 3ppm | 0. 05ppm or less | 0. 05ppm or less |
| Example 4 Agent 1 | 2ppm | 1. 5ppm | 0. 05ppm or less | 0. 05ppm or less |
| Example 4 mixture | 0. 5ppm | 0. 1ppm | 0. 05ppm or less | 0. 05ppm or less |
| Example 5 Agent 1 | 2ppm | 1. 5ppm | 0. 05ppm or less | 0. 05ppm or less |
| Example 5 mixture | 0. 5ppm | 0. 1ppm | 0. 05ppm or less | 0. 05ppm or less |
| Example 6 Agent 1 | 1ppm | 0. 5ppm | 0. 2ppm | 0. 05ppm or less |
| Example 6 Agent 2 | 0. 2ppm | 0. 1ppm | 0. 05ppm or less | 0. 05ppm or less |
| Example 7 Agent 1 | 0. 2ppm | 0. 1ppm | 0. 1ppm | 0. 05ppm or less |
| Example 8 Agent 1 | 1ppm | 0. 5ppm | 0. 4ppm | 0. 05ppm or less |
| | | | | |
| | | | | |
| | | | | |
| | | | | |
| | | | | |

EP 1 430 873 A1

## Fig.10

| | ammonia | amines | hydrogen sulfide | mercaptan |
|---|---|---|---|---|
| | 1 min, 2 strokes | 1 min, 1 stroke | 1 min, 2 strokes | 1.5 min, 4 strokes |
| | pink → yellow | pink → yellow | light yellow → light brown | yellow → red |
| Comparison 1  Agent 1 | 30ppm or more | 100ppm or more | 0. 05ppm or less | 4ppm or more |
| Comparison 1  mixture | 30ppm or more | 100ppm or more | 0. 05ppm or less | 0. 05ppm or less |
| Comparison 2  Agent 1 | 30ppm or more | 100ppm or more | 0. 05ppm or less | 4ppm or more |
| Comparison 2  mixture | 30ppm or more | 100ppm or more | 0. 05ppm or less | 4ppm or more |
| Comparison 3  Agent 1 | 30ppm or more | 100ppm or more | 0. 5ppm | 4ppm or more |
| Comparison 3  mixture | 30ppm or more | 100ppm or more | 0. 05ppm or less | 4ppm or more |
| Comparison 4  Agent 1 | 30ppm or more | 100ppm or more | 0. 05ppm or less | 4ppm or more |
| Comparison 4  mixture | 30ppm or more | 100ppm or more | 0. 05ppm or less | 4ppm or more |
| Comparison 5  Agent 1 | 30ppm or more | 100ppm or more | 0. 05ppm or less | 4ppm or more |
| Comparison 5  mixture | 30ppm or more | 100ppm or more | 0. 05ppm or less | 4ppm or more |
| Comparison 6  Agent 1 | 30ppm or more | 100ppm or more | 0. 05ppm or less | 4ppm or more |
| Comparison 6  mixture | 30ppm or more | 100ppm or more | 0. 05ppm or less | 4ppm or more |
| Comparison 7  Agent 1 | 30ppm or more | 100ppm or more | 0. 2ppm | 4ppm or more |
| Comparison 8  Agent 1 | 30ppm or more | 100ppm or more | 0. 2ppm | 0. 05ppm or less |
| Comparison 9  Agent 1 | 30ppm or more | 100ppm or more | 0. 5ppm | 4ppm or more |
| | | | | |
| | | | | |
| | | | | |
| | | | | |
| | | | | |

EP 1 430 873 A1

<table>
<tr><td colspan="2" align="center"><strong>INTERNATIONAL SEARCH REPORT</strong></td><td>International application No.<br><br>PCT/JP02/08853</td></tr>
</table>

| A. CLASSIFICATION OF SUBJECT MATTER |
|---|
| Int.Cl⁷ A61K7/13, 7/06, 7/09, 7/135 |
| According to International Patent Classification (IPC) or to both national classification and IPC |

| B. FIELDS SEARCHED |
|---|
| Minimum documentation searched (classification system followed by classification symbols)<br>Int.Cl⁷ A61K7/13, 7/06, 7/09, 7/135 |
| Documentation searched other than minimum documentation to the extent that such documents are included in the fields searched |
| Electronic data base consulted during the international search (name of data base and, where practicable, search terms used)<br>CA(STN) |

C. DOCUMENTS CONSIDERED TO BE RELEVANT

| Category* | Citation of document, with indication, where appropriate, of the relevant passages | Relevant to claim No. |
|---|---|---|
| P,X<br>P,Y | JP 2002-193774 A (Kao Corp.),<br>10 July, 2002 (10.07.02),<br>(Family: none) | 1-5,8,10-15<br>6-7,9 |
| P,X | JP 2002-145744 A (Esuteto Kemikaru Kabushiki Kaisha),<br>22 May, 2002 (22.05.02),<br>(Family: none) | 1-15 |
| P,X | JP 2002-68946 A (Hoyu Co., Ltd.),<br>08 March, 2002 (08.03.02),<br>(Family: none) | 1-15 |
| P,X<br>P,Y | JP 2001-328926 A (Hoyu Co., Ltd.),<br>27 November, 2001 (27.11.01),<br>(Family: none) | 1-6,8-15<br>7 |

| ☒ Further documents are listed in the continuation of Box C. | ☐ See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered to be of particular relevance | |
| "E" earlier document but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search<br>21 October, 2002 (21.10.02) | Date of mailing of the international search report<br>05 November, 2002 (05.11.02) |
|---|---|
| Name and mailing address of the ISA/<br>Japanese Patent Office | Authorized officer |
| Facsimile No. | Telephone No. |

Form PCT/ISA/210 (second sheet) (July 1998)

## INTERNATIONAL SEARCH REPORT

| International application No. |
|---|
| PCT/JP02/08853 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y | JP 2001-199851 A (Hoyu Co., Ltd.),<br>24 July, 2001 (24.07.01),<br>(Family: none) | 1-6,8-15<br>7 |
| X<br>Y | JP 2001-122743 A (Yamahatsu Sangyo Co., Ltd.),<br>08 May, 2001 (08.05.01),<br>(Family: none) | 1-8,10-15<br>9 |
| X<br>Y | JP 2001-114657 A (Yamahatsu Sangyo Co., Ltd.),<br>24 April, 2001 (24.04.01),<br>(Family: none) | 1-8,10-15<br>9 |
| X<br>Y | JP 2001-2538 A (Yamahatsu Sangyo Co., Ltd.),<br>09 January, 2001 (09.01.01),<br>(Family: none) | 1-8,10-15<br>9 |
| X<br>Y | JP 2000-336020 A (Yamahatsu Sangyo Co., Ltd.),<br>05 December, 2000 (05.12.00),<br>(Family: none) | 1-8,10-15<br>9 |
| X<br>Y | JP 2000-264822 A (Yamahatsu Sangyo Co., Ltd.),<br>26 September, 2000 (26.09.00),<br>(Family: none) | 1-6,8,10-15<br>7,9 |
| X<br>Y | JP 3-170413 A (Sunstar Inc.),<br>24 July, 1991 (24.07.91),<br>& DE 69008502 C2 | 1-8,10-15<br>9 |
| P,A | JP 2002-145744 A (Esuteto Kemikaru Kabushiki Kaisha),<br>22 May, 2002 (22.05.02),<br>(Family: none) | 1-15 |
| A | EP 712623 A2 (Shiseido Co., Ltd.),<br>22 May, 1996 (22.05.96),<br>& JP 9-71518 A | 1-15 |
| A | JP 6-298626 A (Sanyo Chemical Industries, Ltd.),<br>25 October, 1994 (25.10.94) | 1-15 |

Form PCT/ISA/210 (continuation of second sheet) (July 1998)